# EUROPEAN PATENT APPLICATION

(11) **EP 3 871 502 A1**
(43) Date of publication of application: **01.09.2021**
(21) Application number: 21158995.7
(22) Date of filing: 24.02.2021
(51) Int. Cl.: A01N 59/00, A01N 59/20, A01N 65/28, A01N 65/08, A01P 3/00

(54) **ANTIFUNGAL COMPOSITIONS AND METHODS OF USING SAME**

(30) Priority: 25.02.2020 US 202062981289 P
(71) Applicant: Columbus LTACH, LLC, Newark, NJ 07107 (US)
(72) Inventor: KOHUT, Karli, Parsippany, NJ 07054 (US); MEJIA, Milka, North Bergen, NJ 07047 (US); KRUTY, Olga, Lyndhurst, NJ 07071 (US)
(74) Representative: FRKelly

(57) **Abstract**

Anti-fungal compositions, methods of making same, uses thereof, and kits including one or more composition(s). A composition may include optionally, copper sulfate; a copper metal component; and one or more organic antifungal component(s). A copper metal component may be a copper powder. An organic antifungal component may be tea tree oil, jojoba oil, or the like, or a combination thereof. A composition may also include one or more carbon source(s) and/or one or more essential oil(s). A composition may be used to treat fungal infections or treat medical equipment, medical devices, food-handling implements, or the like.

## Description

### CROSS-REFERENCE TO RELATED APPLICATIONS

This application claims priority to U.S. Provisional Patent Application No. 62/981,289, filed February 25, 2020, the disclosure of which are incorporated herein by reference.

### SUMMARY OF THE DISCLOSURE

The present disclosure provides antifungal compositions. The present disclosure also provides uses of the compositions. In various examples, the present disclosure demonstrates how copper metal and, optionally, copper ions, in combination with other substances containing antifungal properties, can, for example, suppress and destroy, fungi, such as, for example, Candida auris colonies. The present disclosure also provides effective treatment plans, for example, to treat and de colonize Candida auris from an individual (e.g., a patient's skin), equipment used, the environment, or a combination thereof. It was found that compositions of the present disclosure were effective in treating fugal infections, such as, for example, Candia auris (e.g., eliminating and suppressing any new growth of Candida auris and destroying colonies already present).

In an aspect, the present disclosure provides antifungal compositions. The compositions comprise copper metal and copper ions, which are optional, and one or more antifungal compound(s). The compositions may be used to treat fungal infections. In various examples, an antifungal composition comprises: optionally, copper sulfate; a copper metal component (e.g., copper particles); one or more organic antifungal component(s), optionally, one or more carbon source(s), and, optionally, one or more essential oil(s). One or more or all of the component(s) or a composition may be anhydrous or substantially anhydrous. A composition may comprise one or more additional component(s). Non-limiting examples of additional components include carbon source(s) (e.g., carbon powders (such as, for example, activated charcoals and the like), and the like) and oils, which may be essential oil(s), waxes, and the like.

A composition may be used to treat an individual (e.g., an individual having or suspected of having a fungal infection), inanimate objects, such as, for example, medical equipment, medical devices, implements used in food handling, or the like, or combinations thereof.

Compositions can be made by various methods. Non-limiting examples of methods of making compositions of the present disclosure are provided herein.

In an aspect, the present disclosure provides pharmaceutical compositions comprising one or more composition(s) of the present disclosure. In various examples, a pharmaceutical composition comprises one or more composition(s) of the present disclosure and one or more additional pharmaceutical component(s), which may be one or more pharmaceutically acceptable excipient(s).

In an aspect, the present disclosure provides articles of manufacture. Non-limiting examples of articles of manufacture include medical equipment, medical devices, implements used in food handling, and the like. An article of manufacture comprises one or more composition(s) and/or one or more pharmaceutical composition(s) of the present disclosure. The one or more composition(s) and/or one or more pharmaceutical composition(s) may be disposed on at least a portion of or all of one or more surface(s) of the article of manufacture and/or impregnated or infused in at least a portion thereof.

In an aspect, the present disclosure provides methods of treating fungal infections. The methods may use one or more composition(s) of the present disclosure. In various examples, a method of inhibiting or preventing the growth of and/or killing one or more fungi comprises: contacting at least a portion of or all of a surface with one or more composition(s) of the present disclosure. In various examples, a method of treating or preventing a fungal infection in an individual in need thereof (e.g., an individual having a or suspected of having a fungal infection) comprises administering to the individual a therapeutically effective amount of one or more composition(s) of the present disclosure. A method may further comprise testing for the presence of the one or more fungi after one or more of the contacting(s). Non-limiting examples of testing methods are described herein. In various examples, a method of treating a fungal infection comprises administering a therapeutically effective amount of one or more composition(s) of the present disclosure to an individual in need thereof. A method may further comprise identifying an individual in need of treatment. In various example, the fungal infection is caused by one or more fungi chosen from those of the genus Candida. Non-limiting examples of Candida fungi include, Candida albicans, Candida lusitaniae, Candida kruseii, Candida glabrata, Candida parapsilosis, Candida tropicalis, Candida guilliermondii, and the like, and combinations thereof.

In an aspect, the present disclosure provides kits. A kit comprises one or more composition(s) of the present disclosure and instructions for use of the composition(s). The instructions may be instructions to carry out one or more method(s) of the present disclosure. A kit optionally includes a label or insert including a description of the composition(s) and/or the individual component(s) of the composition(s) (e.g,, type, amounts, doses, and the like), instructions for use of the composition(s) and/or the individual component(s) of the composition(s).

### BACKGROUND OF THE DISCLOSURE

The increase in fungal infections and resistance to traditional therapies is a significant public health threat worldwide. These infections are becoming more common, in part due to an increase in those susceptible to such infections. This subpopulation includes, for example, the immunocompromised: individuals undergoing chemotherapy, those receiving immunosuppressive drugs following transplantations, and those immunosuppressed due to diseases, such as, for example, AIDS or malignancies, etc.

Candida auris poses a threat to the general population, specifically for hospitalized and immunocompromised patients. Preventable deaths, financial and logistical concerns, have been reported by the media and the Centers for Disease Control, (CDC) which have resulted from to Candida auris infections and colonizations.

According to the CDC, an estimated 35,000 new cases of Candida auris have been identified in the United States among hospitalized patients with 1,700 of them resulting in death. This number is still rising worldwide. With no known cure and the known resistance of Candida auris to common antifungal treatments, Candida auris poses a global urgent threat. Recently reported new cases are heavily focused in NY, NJ, CT, and CA. However, this fungus is expected to continue to spread throughout the nation.

### BRIEF DESCRIPTION OF THE FIGURES

The patent or application file contains at least one drawing executed in color. Copies of this patent or patent application publication with color drawing(s) will be provided by the Office upon request and payment of the necessary fee.

For a fuller understanding of the nature and objects of the disclosure, reference should be made to the following detailed description taken in conjunction with the accompanying figures.

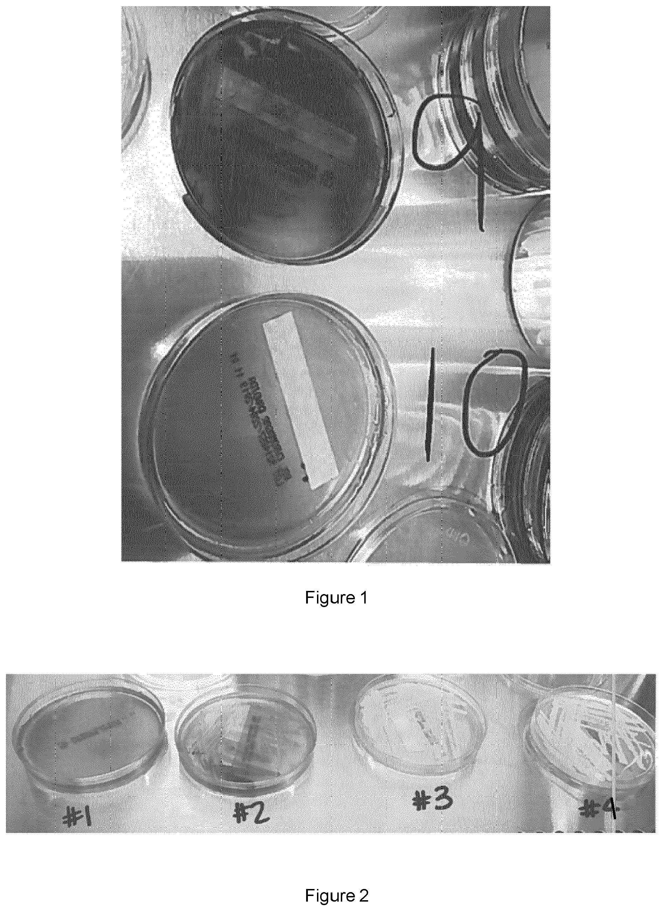

Figure 1 shows an image of a sample of copper powder, copper sulfate, and tea tree oil. Plate #9 shows copper powder with hydrogel where growth was minimal but still evident. After 2 days, tea tree oil was added to the plate, and the growth was minimal but still evident. It was obverved that water increases the growth of candida auris. Plate #10 shows complete suppression and destruction of Candida auris. The three ingredients used were copper sulfate, copper powder, and tea tree oil.
Figure 2 shows an image of four samples: #1 Teatree, copper sulfate, copper powder; #2 Teatree, copper sulfate, copper powder, charcoal; #3 No treatment; and #4 No treatment. #1 and #2 show the formulas were effective in killing Candida auris. #3 and #4 are controls shown with no treatment.

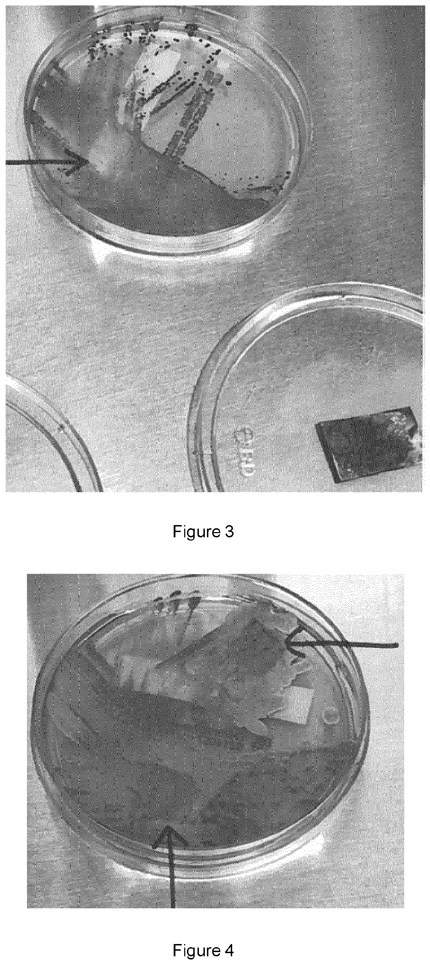

Figure 3 shows an image of a copper foil square after one day. Suppression of growth under the area where the copper foil was placed is shown.
Figure 4 shows an image of copper foil squares. Suppression of growth under the areas where copper foil squares were placed is shown.

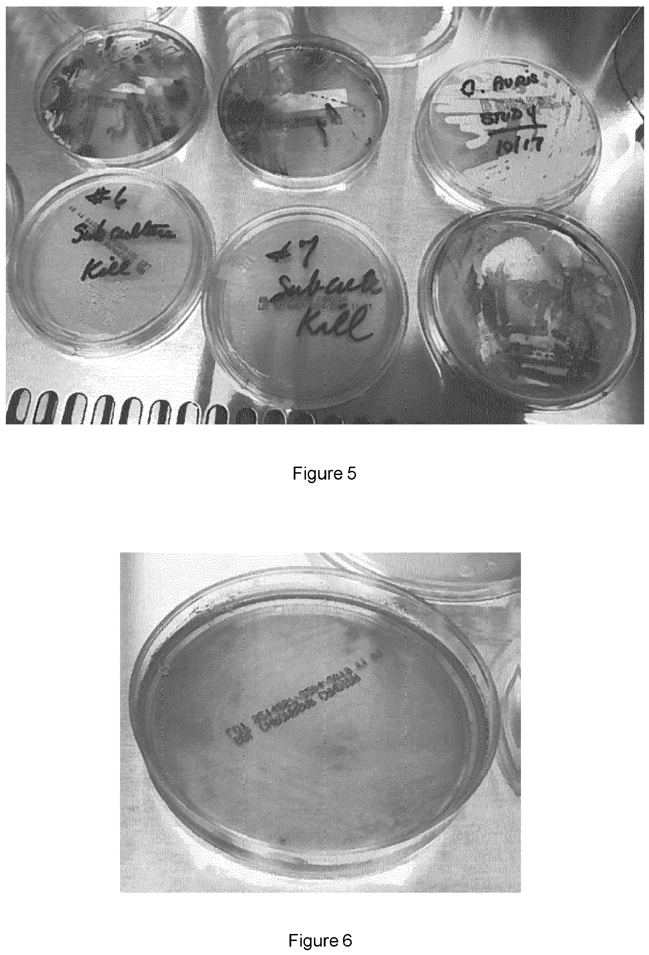

Figure 5 shows an image of two samples: #6 Teatree oil, copper sulfate, copper powder (subculture taken from treated areas shows no growth); #7 Jojoba oil, copper sulfate, copper powder (subculture taken and show no growth).
Figure 6 shows an image of a sample of jojoba oil, copper sulfate, copper powder plus roasted garlic. There was no growth.

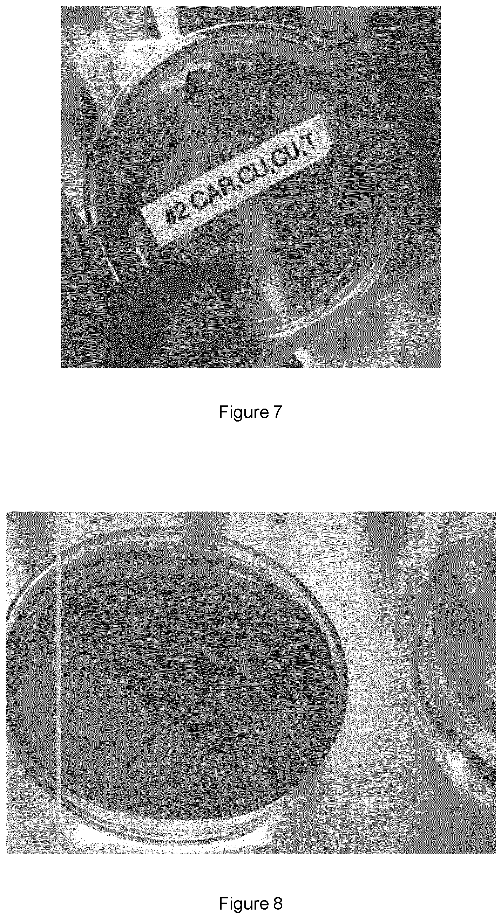

Figure 7 shows an image of a sample where there is no growth after the addition of tea tree oil, copper sulfate, copper powder, and charcoal.
Figure 8 shows an image of a sample where tea tree oil and copper sulfate alone did not destroy all growth, but after the addition of copper powder, no Candida auris grew. It was discovered that additional copper may be important in the formula.
Figure 9 shows examples of images that may provide guidance for reading a plate in a testing method.

### DETAILED DESCRIPTION OF THE DISCLOSURE

Although claimed subject matter will be described in terms of certain embodiments and examples, other embodiments and examples, including embodiments and examples that do not provide all of the benefits and features set forth herein, are also within the scope of this disclosure. Various compositional, structural, logical, and process step changes may be made without departing from the scope of the disclosure.

Ranges of values are disclosed herein. The ranges set out a lower limit value and an upper limit value. Unless otherwise stated, the ranges include all values to the magnitude of the smallest value (either lower limit value or upper limit value) and ranges between the values of the stated range.

Percent by weight (% by weight) as used herein, unless otherwise indicated, means percent by weight based on the total composition weight or the total weight of the copper sulfate (if present), copper metal, and organic antifungal component(s) or the total weight of the copper sulfate (if present), the copper metal, the organic antifungal component(s), the carbon source(s) (if present), and, one or more essential oil(s) (if present).

The present disclosure provides antifungal compositions. The present disclosure also provides uses of the compositions.

In various examples, the present disclosure demonstrates how copper metal and, optionally, copper ions, in combination with other substances containing antifungal properties, can, for example, suppress and destroy, fungi, such as, for example, Candida auris colonies. The present disclosure also provides effective treatment plans, for example, to treat and decolonize Candida auris from an individual (e.g., a patient's skin), equipment used, the environment, or a combination thereof. It was found that compositions of the present disclosure were effective in treating fugal infections, such as, for example, Candia auris (e.g., eliminating and suppressing any new growth of Candida auris and destroying colonies already present).

In an aspect, the present disclosure provides antifungal compositions. The compositions comprise copper metal and copper ions, which are optional, and one or more antifungal compound(s). The compositions may be used to treat fungal infections. Non-limiting examples of compositions are provided herein.

In various examples, an antifungal composition comprises: optionally, copper sulfate; a copper metal component (e.g., copper particles); one or more organic antifungal component(s), optionally, one or more carbon source(s), and, optionally, one or more essential oil(s). In various examples, the amounts (e.g., weight percentages) of copper sulfate, if present, copper metal component, one or more organic antifungal component(s), optionally, one or more carbon source(s), optionally, one or more essential oil(s), and, optionally, additional component equals 100%. It may be desirable that both the copper sulfate and copper metal component (e.g., copper powder) are each a fine powder (e.g., having one or more or all of the flow and/or rheological characteristic(s) of cornstarch or talcum powder, examples of which are known in the art and commercially available). In various examples, the copper sulfate is present at 55 to 68 percent by weight, including all 0.1 percent by weight values and ranges therebetween. In various examples, the copper metal component(s) (e.g., copper powder(s)) is/are present at 30 to 44 percent by weight, including all 0.1 percent by weight values and ranges therebetween. In various examples, the organic antifungal component(s) is/are present at 0.5 to 2 percent by weight (individually or in the aggregate, if more than one such component is present), including all 0.1 percent by weight values and ranges therebetween. In various examples, the copper sulfate is present at 55 to 68 percent by weight, including all 0.1 percent by weight values and ranges therebetween, and/or the copper metal component(s) (e.g., copper powder(s)) is/are present at 30 to 44 percent by weight, including all 0.1 percent by weight values and ranges therebetween, and/or the organic antifungal component(s) is/are present at 0.5 to 2 percent by weight (individually or in the aggregate, if more than one such component is present), including all 0.1 percent by weight values and ranges therebetween. One or more or all of the component(s) or a composition may be anhydrous or substantially anhydrous.

By "substantially anhydrous" it is meant that the amount of water in the composition does not increase the growth of one or more fungi relative to a composition with the same components that is anhydrous and/or the composition has 1% or less by weight, 0.5% or less by weight, 0.1% by weight, or no observable (by one or more method(s) known in the art) water. In the case of a water-based composition (e.g., a tincture or a slurry) it may be desirable that the composition does not have a pH that forms sulfuric acid, in the case of a compostion that comprises copper sulfate.

It may be desirable that the amount of copper sulfate and copper powder is greater than the sum of the additional components of the composition. In various examples, the ratio of the copper sulfate and copper powder to the additional components of the composition is 2:1 or greater or 3:2 or greater. In various other examples, the ratio of the copper sulfate and copper powder to the additional components of the composition is 3:2 to less than 4:2.

Without intending to be bound by any particular theory, it is considered that the copper powder and copper sulfate, if present, are not absorbed into the skin, but remain on the surface of the skin to kill the fungi (e.g., Candida auris).

In various examples, a composition comprises or is
- 10 grams copper sulfate, 5 grams copper powder, 100mL jojoba oil, and 50 mL tea tree oil;
- 10 mL tea tree oil, 8 grams copper sulfate, and 5 grams copper powder;
- 10 grams copper sulfate, 6 grams copper powder, 180 mL jojoba oil;
- 10 grams copper sulfate, 5 grams copper powder, 100mL jojoba oil, 50 mL tea tree oil,
- 0.5 mg of fermented roasted garlic oil;
- 5 grams copper powder; 50 mL tea tree oil, 0.5 capsule of activated charcoal; or
- 180mL of jojoba oil, tree oil, or a combination thereof, 80,000 mg of copper sulfate, and 50,000 mg of copper powder, optionally, 50 mg of activated charcoal, and, optionally, 50 mL of optional essential oil (which may have anti-fungal properties).
In these examples, one or more or all of the component(s) may be anhydrous or substantially anhydrous.

It may be desirable that a composition comprises jojoba oil and/or tea tree oil. In various examples, the one or more organic antifungal component(s) of the composition is/are jojoba oil and/or tea tree oil. In various other examples, the one or more organic antifungal component(s) of the composition is/are at least jojoba and/or tea tree oil.

In various examples, the copper sulfate is present at 55 to 68 percent by weight. In various examples, the copper metal component(s) (e.g., copper powder(s)) is/are present at 30 to 44 percent by weight (individually or in the aggregate, if more than one such component is present). In various examples, the organic antifungal component(s) is/are present at 0.5 to 2 percent by weight (individually or in the aggregate, if more than one such component is present).

A composition may comprise one or more additional component(s). Non-limiting examples of additional components include carbon source(s) (e.g., carbon powders (such as, for example, activated charcoals and the like), and the like) and oils, which may be essential oil(s), waxes, and the like, and combinations thereof. It may be desirable that the carbon source(s) is/are nitrate inhibitors. It is desirable that the essential oil(s) exhibit an antifungal property. In various examples, a composition may comprise one or more additional agent(s) for treatment of, for example, vaginal, fungal, viral, or other bacterial or parasitic infections, or the like. Such additional agents include, but are not limited to, antibiotics and anti-viral agents, and chemical compounds that act as biocides and/or antiseptic agents. Additional agents may also include, but are not limited to, soothing agents or compositions that comprise, for example, one or more anti-irritant and/or anti-inflammatory agent(s), such as, for example, hydrocortisone or related compounds, emollients, anti-hemorrhagic, hemostatic, anti-allergic agents, and the like. In various examples, 0.01 to 1 percent by weight of the copper sulfate (individually or in the aggregate, if more than one such component is present) is replaced by one or more additional component(s), which may be essential oils(s) . In various examples, the carbon source(s) is/are present at 0.5 to 1 percent by weight (individually or in the aggregate, if more than one such component is present), including all 0.1 weight percent values and ranges therebetween, and/or the essential oil(s) is/are present at 0.01 to 1 percent by weight (individually or in the aggregate, if more than one such component is present), including all 0.1 weight percent values and ranges therebetween. One or more or all of the additional component(s) may be anhydrous or substantially anhydrous.

Non-limiting examples of essential oils include lavender oil, white sage oil, marjoram oil, neroli oil, patchouli oil, peppermint oil, palmarosa oil, myrrh oil, thyme oil, cassia oil, savory oil, lemongrass oil, citronella oil, coriander seed oil, eucalyptus oil, sweet fennel oil, ho wood oil, kanuka oil, rosemary oil, rosewood oil, vertiver oil, oregano oil, and the like, and combinations thereof. Without intending to be bound by any particular theory, it is considered that addition of essential oil(s) may provide a composition with desirable scent or smell.

The compositions may have various forms. In a non-limiting example, the composition is in the form of a cream, suspensions or dispersions, oils, balms, lotions, gels, cream gels, liniments, serums, films, ointments, sprays or aerosols, other forms of coating, or any multiple emulsions, slurries or tinctures. Non-limiting examples of compositions include nail polishes, nail fungus treatments, hair removal waxes, make-up removers, and the like. It may be desirable that any of these compositions is substantially anhydrous or anhydrous.

A composition exhibits one or more antifungal property(ies). In various examples, a composition inhibits or prevents the growth of and/or kills one or more fungi. Non-limiting examples of fungi include Candida (such as, for example, c. auris, c. albicans, c. parapsilosis, c. lusitaniae, c. kruseii, c. glabrata, c. tropicalis, c. guilliermondii, and the like, and combinations thereof) and the like, and combinations thereof.

A composition may be used to treat an individual (e.g., an individual having or suspected of having a fungal infection), inanimate objects, such as, for example, medical equipment, implements used in food handling, or the like, or combinations thereof. Non-limiting examples of medical equipment include: medical instruments, medical devices, beds and related equipment, such as, for example, bed frames, bed rails, call bells, and the like, medical clothing, such as, for example, gowns, caps, masks, gloves, socks, shoe covers, and the like, bedding materials, such as for example, mattresses, sheets, blankets, pillows, pillow cases, and the like, phones, and the like (e.g., anything typically found in a patient's room). Other non-limiting examples of medical equipment include cotton swabs infused with one or more composition(s), personal razor moisturizing/protective strips, eyebrow threaders, stethoscope covers, medical equipment covers, EKG leads, household air vent filters, home heating and air conditioning filters, fish tank filters *, shoe liners and insoles *, arch supports, * door handle/faucet handle covers, cart handles, and the like, dental flossers and picks, and the like. *= copper and charcoal only/no oils.

In various examples, a medical device is an implantable medical device or other implanted surgical object, which may be either permanent or temporarily implanted. Non-limiting examples of medical devices include catheters (such as, for example, indwelling catheters, and the like), surgical implants of any kind, and the like. Other non-limiting examples of medical devices include copper -coated indwelling foley catheters, and intravenous catheters, vacuum assisted wound closure devices, sterile central line IV/arterial dressing patches and coverings, copper-coated pacemakers or portacatheters, and the like.

Compositions of the present disclosure may be incorporated into a coating on a and/or impregnated or infused in a tampon or tampon-like device, sponge, suppository, or other absorbent material.

In various examples, the composition(s) are incorporated into a tampon-fitting device. In certain examples, the composition is provided in association with fibrous material, which may be synthetic or natural (such as, for example, rayon, polyester, cotton, and the like, and combinations thereof). The compositions may be incorporated into sanitary napkins, adult diapes and and bladder leakage pads and underwear. Thus, the disclosure includes a variety of devices that can be used as applicators of the compositions described herein.

In various examples, the present disclosure disclosure provides a vaginal tampon, vaginal ring, vaginal cup, vaginal tablet, vaginal sponge, a vaginal bioadhesive tablet, a vaginal lubricant, a condom, or a modified female hygiene or other vaginal health care product, such as, for example, prescription and over-the-counter antifungal products that treat and/or cure vaginal yeast infections, or bacterial vaginosis, but that have been adapted to include one or more composition(s) of the present disclosure for prophylaxis and/or therapy of a fungal infection. Applicators that are provided with female hygiene or vaginal health care products can be adapted for intravaginal administration of one or more composition(s) of the present disclosure. The delivery composition may be formulated to adhere to and act directly on the vaginal epithelium and/or mucosa, and such formulations can be provided if desired without components that promote the transport or transfer of one or more component(s) of the composition(s) through the vaginal wall. Thus, in certain approaches one or more component(s) of the composition(s) does not enter the circulatory system of the individual.

In various examples, the compositions do not comprise water (e.g., are anhydrous) and/or Group I cation salt(s).

Compositions can be made by various methods. As an illustrative example, a composition is made by:
- grinding copper powder finely (e.g., such that powder has flow properties of cornstarch or talcum powder);
- grind copper sulfate crystals finely (e.g., such that powder has flow properties of cornstarch or talcum powder);
- weigh and add (e.g., using a pipette) each non-optional component to a clean cup, which may be a sterile cup;
- add any optional components;
- mix vigorously. The mixture should remain as a suspension for use. (If needed, shake or mix immediately before application).

In an aspect, the present disclosure provides pharmaceutical compositions comprising one or more composition(s) of the present disclosure. Non-limiting examples of pharmaceutical compositions are provided herein.

In various examples, a pharmaceutical composition comprises one or more composition(s) of the present disclosure and one or more additional component(s), which may be one or more additional pharmaceutical component(s), such as, for example, one or more pharmaceutically acceptable excipient(s). Non-limiting examples of suitable excipients are well known in the art and are described in, for example, Garg S., et al. Compendium of Pharmaceutical Excipients for Vaginal Formulations, Pharmaceutical Technology DRUG DELIVERY (2001), p. 14-24, from which the description of excipients is incorporated herein by reference. Other components may be present in a pharmaceutical composition, such as, for example, anti-oxidants, chelating agents, preservatives, oils (e.g., coconut oil and the like), waxes (e.g., beeswax and the like), alcohols, surfactants, emulsifiers, viscosity building agents, solvents, moisturizing agents, solubilizers and the like, antiseptic chemicals, and other specific components, such as, for example, propylenes, polypropylenes, polysorbates, and a variety of simple polyol compounds, i.e., glycerol. The relative quantities of such components may vary according to the desired nature and consistency of the composition, including creams, ointments, waxy suppositories, gelatin capsules, anhydrous polymeric suppositories, and the like.

In various examples, a composition (which may be a pharmaceutical composition) is a balm and comprises beeswax. In various other examples, a composition (which may be a pharmaceutical composition) comprises an oil, such as, for example, coconut oil. Such a composition may be an lotion or a cream. In various other examples, a pharmaceutical composition comprises petroleum jelly (e.g., a mixture of waxes and mineral oil) or the like. Without intending to be bound by any particular theory it is considered that such compositions may provide desirable coating of the skin and filling of the compositon into the joint creases, inner elbows, groin, underarms, anus, etc.

A composition (which may be a pharmaceutical composition) may be an all-natural compositon (e.g., the compositon does not comprise any component that requires a preservative, for example, to extend shelf life).

In an aspect, the present disclosure provides articles manufacture. An article of manufacture comprises one or more composition(s) and/or one or more pharmaceutical compositon(s) of the present disclosure. The one or more composition(s) and/or one or more pharmaceutical composition(s) may be disposed on at least a portion of or all of one or more surface(s) of the article of medical equipment. One or more surface(s) of the article of manufacture may be exterior surface(s), interior surface(s), or the like, or a combination thereof. An article of manufacture may comprise one or more composition(s) and/or one or more pharmaceutical composition(s) of the present disclosure impregnated or infused therein. An article of manufacture may be an article of medical equipment, a medical device, implements used in food handling, or the like.

Non-limiting examples of articles of manufacture, which may be articles of medical equipment, include: medical instruments, medical devices, beds and related equipment, such as, for example, bed frames, bed rails, call bells, and the like, medical clothing, such as, for example, gowns, caps, masks, gloves, socks, shoe covers, and the like, bedding materials, such as for example, mattresses, sheets, blankets, pillows, pillow cases, and the like, phones, and the like (e.g., anything typically found in a patient's room). Other non-limiting examples of articles of manufacture include cotton swabs infused with one or more composition(s) and/or pharmaceutical composition(s), personal razor moisturizing/protective strips, eyebrow threaders, stethoscope covers, medical equipment covers, EKG leads, air vent filters (e.g., household air vent filters and the like), heating and air conditioning filters (home heating and air conditioning filters and the like), fish tank filters *, shoe liners and insoles *, arch supports * door handle/faucet handle covers, cart handles and the like, dental flossers and picks, and the like. *= copper and charcoal only/no oils.

Non-limiting examples of medical equipment include tampons, tampon-like devices, sponges, suppositories, or other absorbent materials. One or more composition(s) and/or one or more pharmaceutical composition(s) may be disposed on at least a portion of or all of one or more surface(s) of a tampon. The one or more surface(s) of the tampon may be exterior surface(s), interior surface(s), or the like, or a combination thereof. The one or more composition(s) and/or one or more pharmaceutical composition(s)ompositions may be incorporated into or be present as a coating on the tampon or tampon-like device, sponge, suppository. An article of medical equipment, which may comprise or be an absorbant material, may comprise one or more impregnated composition(s) and/or one or more pharmaceutical composition(s).

In various examples, a tampon-fitting device comprises one or more impregnated composition(s) and/or one or more pharmaceutical composition(s). In certain examples, the composition is associated with (e.g., disposed on at least a portion of or all of one of one or more surface(s) of a fibrous material, which may be synthetic or natural (such as, for example, rayon, polyester, cotton, and the like, and combinations thereof). In non-limiting examples, medical devices include sanitary napkins, adult diapes and and bladder leakage pads and underwear comprising one. In other non-limiting examples, examples of medical devices include vaginal tampons, vaginal rings, vaginal cups, vaginal tablets, vaginal sponges, vaginal bioadhesive tablets, condoms, modified female hygiene or other vaginal health care products, such as, for example, prescription and over-the-counter antifungal products that treat and/or cure vaginal yeast infections, or bacterial vaginosis, comprising one or more composition(s) and/or pharmaceutical composition(s) of the present disclosure.

In various examples, a medical device is an implantable medical device or other implanted surgical object, which may be either permanent or temporarily implanted. Non-limiting examples of medical devices include catheters (such as, for example, indwelling catheters, and the like), surgical implants of any kind, and the like. Other non-limiting examples of medical devices include copper -coated indwelling foley catheter, and intravenous catheters, vacuum assisted wound closure devices, sterile central line IV/arterial dressing patches and coverings, copper-coated pacemakers or portacatheters, and the like.

In an aspect, the present disclosure provides methods treating fungal infections. The methods may use one or more composition(s) of the present disclosure. In a method, reference to one or more composition(s) includes composition(s), pharmaceutical composition(s), or a combination thereof. Non-limiting examples of methods treating fungal infections are provided herein.

In various examples, a method of inhibiting or preventing the growth of and/or killing one or more fungi comprises: contacting at least a portion of or all of a surface with one or more composition(s) of the present disclosure. One skilled in the art would appreciate that the amount of composition(s) used will depend on the size of the surface (e.g., individual). As an illustrative example, approximately twelve ounces per application is contacted twice a day.

The surface may be part or all of an article of medical equipment (e.g., all or part of the exterior surface and/or interior surface of the medical equipment) and/or at least a portion or all of the skin of an individual (e.g., a human or non-human mammal). This may be referred to as a topical treatment. One skilled in the art would appreciate that the amount of composition(s) used will depend on the size of the individual. As an illustrative example, approximately twelve ounces per application is contacted twice a day.

In various examples, a method of treating or preventing a fungal infection in an individual in need thereof (e.g., an individual having a or suspected of having a fungal infection) comprises administering to the individual a therapeutically effective amount of one or more composition(s) of the present disclosure. In the case of an individual, at least a portion of or all of the composition(s) may be absorbed into to the skin of the individual. One skilled in the art would appreciate that the absorption time will vary depending on the individual. As an illustrative example, typically, the absorption takes about an hour.

The contacting may require two or more individual contacting treatments achieve the desired results. As an illustrative example, in the case where two or more individual contacting treatments are used, the individual contacting treatments are repeated every 10 to 14 hours (e.g., about every 12 hours), including all 0.1 hour values and ranges therebetween and/or the contacting is carried out over a period of 14 days or less. The individual may be retested after conclusion of the contacting and, if appropriate, the contacting may be repeated.

It may be desirable that the individual is hairless or substantially hairless. In various examples, substantially all or all of the hair, if present, of the human or non-human animal is removed (e.g., mechanically or chemically) prior to contact with the composition(s). By "substantially all of the hair" it is meant that the amount of hair does not materially effect the treatment or prevention of the fungal infection by the composition(s) or pharmaceutical composition(s).

It may be desirable that the individual is in contact isolation during the contacting. The individual may be in contact isolation for a desired period of time after the contacting(s).

It may be desirable that the surface (e.g., the surface of the individual, which may be the contacted surface(s) of the individual is not exposed to liquid water immediately before the contacting and/or during the contacting and/or for a desired period of time after the contacting(s). Non-limiting examples of exposure to liquid water include bathing and the like.

A method may comprise contacting the human or non-human animal with a waterless/water-free composition (e.g., a cleanser) prior to the contacting. Without intending to be bound by any particular theory, it is considered that the waterless/water-free composition removes at least a portion of or all of the sweat, product, or moisture, or a combination thereof, from the surface (e.g., skin) of the human or non-human animal.

As part of a method, it may be desirable to treat at least a portion or all of the surfaces that the individual may contact (e.g., are in proximity to or in the room in which the individual are treated). A method may comprise treating at least a portion or all of the surfaces that the individual may contact (e.g., are in proximity to or in the room in which the individual are treated) with one or more composition(s) of the present disclosure. This contacting may be prior to (e.g., immediately prior to) contacting the individual. The surfaces may be a portion of or all of the medical equipment used to during the contacting and/or treating.

A method may further comprise testing for the presence of the one or more fungi after one or more of the contacting(s). The testing can be carried out by methods known in the art. The testing may be carried out by culture-based methods or culture-independent methods. For example, the testing is carried out by protocols published (e.g., for testing for Candida auris) by the Center for Disease Control (CDC) and the New Jersey State Department of Health. Examples of testing protocols are available at www.CDC.gov. (e.g., at candidaauris@cdc.gov') and NJDOH.

A non-limiting example of a testing method follows. The following is a process/procedure/protocol for obtaining and processing swabs to assess for *C. auris* colonization:

### Materials

- Culture collection and transport system used to swab a patient
- Rayon tip swabs (e.g., Fisherfinest Amies Charcoal bacteriology culture collection and transport system; Fisher healthcare, Ontario, Canada)
- CHROMagar *Candida* Chromogenic agar (e.g., *Candida* CHROMagar available from Becton Dickinson, San Jose, CA)
- 10 µl transfer loops
- Stationary Incubator (37°C)

Procedure for patient swab processing to isolate *Candida auris* via direct plating. Swabs from patients will likely contain a complex community of microorganisms. It is important, and may be necessary, to isolate *C. auris* from this community in order to obtain accurate species identification. The following describes steps to isolate *C. auris* from patient swabs. It is desirable that the following steps are performed in a biological safety cabinet to ensure safety and sterility.

It is desirable to use media that has been quality-controlled for this procedure and perform negative controls. Positive controls are optional.
- Label a CHROMagar *Candida* Chromogenic agar plate for each sample and ensure that all information matches specimen submission form. Include 2 sterile unopened swabs culture transport systems and 2 extra plates for the controls (described below).
- Open the swab culture transport systems and remove the swab applicator. Streak the swab across one quadrant of the CHROMagar *Candida* plate rolling the swab to ensure contact of all sides of the swab with the plate.
- Use the 10 µl transfer loops to streak out from the swab-inoculated quadrant for isolation on CHROMagar *Candida* plates.
- If using a positive control, inoculate the positive control swab transport system by touching the swab to a single colony of *C. auris* and replacing the swab in the transport system. Then, directly transfer the positive swab to a CHROMagar *Candida* agar plate and use a loop to streak for isolation using the same method described above.
- Open and replace the negative culture swab transport system. Directly transfer the negative swab to a CHROMagar *Candida* plate using the same method described above (second and third steps).
- Incubate CHROMagar *Candida* plates at 37°C. Optimum growth temperature for *C. auris* is 37-40°C. Hold all cultures for 7 days of incubation on CHROMagar *Candida* agar plates. Check for colonies after 48 hours, but continue incubating and re-examining negative plates daily for 7 days.
- Once visible growth is detected, use the 10 µl transfer loops to streak all white, pink, and red colonies for isolation on CHROMagar *Candida* plates. Streaking all white, pink and red colonies is critical, as *C. auris* cannot be distinguished from other *Candida* by colony morphology. Incubate at 37°C, monitoring the plates daily and re-streaking if needed to confirm isolation.
- Identify the species of all isolates from the step above. bioMerieux VITEK 2 with on 8.01 update
- All confirmed *C. auris* isolates should be reported to state and local health departments and CDC at candidaauris@cdc.go
   *Candida auris* from patient samples has the tendency to display a delayed revival or slow growing phenotype and it is not uncommon to see CFUs arise after 5-7 days of incubation for these samples. However, once colonies present themselves they can be streaked out for isolation and grow as normal.

Quality Control. Negative Control may be run every time with patient sample, including, but not limited to, following negative control strains: *Candida albicans* ATCC 6019 - Light to medium green colonies; *Candida tropicalis* ATCC 1369 - Gray blue to blue-greenish or metallic blue colonies with or without violet halos in the surrounding medium.

The following is an example of a flowchart for a testing method:

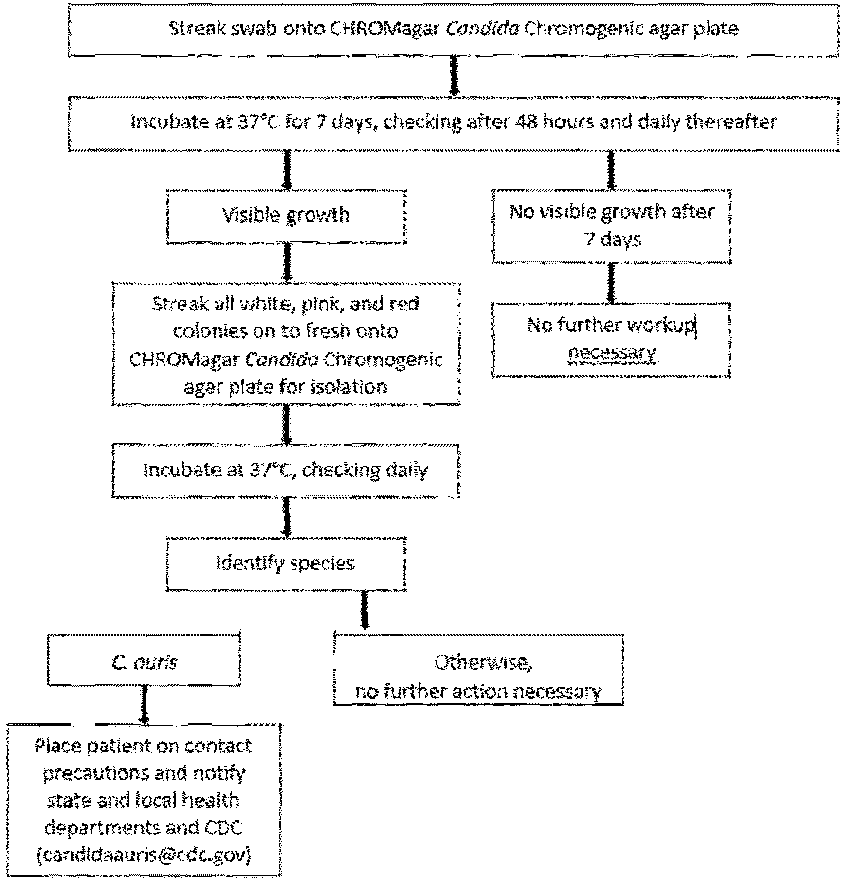

### Media Quality Control:

Storage: On receipt, plates will be stored in the dark at 2 to 8° C, in their original sleeve wrapping until just prior to use. Avoid freezing and overheating. The plates may be inoculated up to the expiration date (see package label) and incubated for the recommended incubation times. Plates from opened stacks of 10 plates can be used for one week when stored in a clean area at 2 to 8° C.

Materials: BBL CHROMagar Candida Medium and laboratory equipment as required.

Quality Control Procedure: Inoculate representative samples with the quality strains onto the surface of the medium. Incubate the plates aerobically for 20 to 48 hours at 35 +/- 2° C. Note that an incubation of 42 hours is required for full color. Minimize exposure to light both before and during incubation.

Results. After incubation, read the plates on a white background following the below table for colony differentiation from color

| **Strains** | **BBL CHROMagar Candida Medium** |
|---|---|
| *Candida albicans* ATCC 60193 | Growth good to excellent; light to medium green colonies |
| *Candida albicans* ATCC 10231 | Growth good to excellent; light to medium green colonies |
| *Candida krusei* ATCC 34135 | Growth good to excellent; light rose to pink, large tlat colonies with a whitish border |
| *Candida tropicalis* ATCC 1369 | Growth good to excellent; gray blue to blue-greenish or metallic blue colonies with or without violet halos in the surrounding medium |
| *Candida tropicalis* ATCC 9968 | Growth good to excellent; gray-blue colonies with or without violet halos |
| *Pseudomonas aeruginosa* ATCC 27853 | Inhibition partial to complete |
| Uninoculated | Colorless to light beige, transparent |

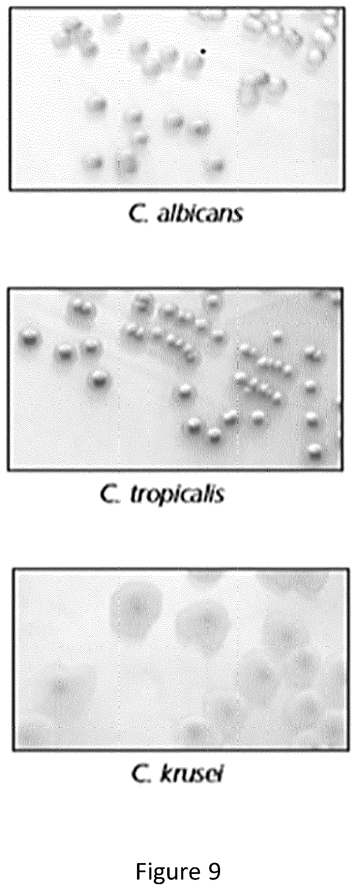

Non-limiting examples of images that may provide guidance for reading a plate are shown in Figure 9.

CHROMagar Candida Medium Quality Control: Media QC should be done on each new shipment and lot.

Safety considerations. *C. auris* can survive for weeks on plastic surfaces, has reduced susceptibility to quaternary ammonia disinfectants, and can colonize skin of healthy individuals.

Therefore, strict BSL2 laboratory safety precautions should be followed when working with this organism. Specifically, it is recommended that cultures are processed within BSL2 biosafety cabinet, gloves and lab coats are required, and strong hand hygiene is enforced. The use of disinfectants with sporicidal claim, such as, for example, freshly made 10% bleach, are recommended for decontaminations after working with *C. auris* cultures.

In various examples, a method of treating a fungal infection comprises administering a therapeutically effective amount of one or more composition(s) of the present disclosure to an individual in need thereof. A method may further comprise identifying an individual in need of treatment.

In some examples, the individual is not immunocompromised. In some examples, the individual is immunocompromised. In some examples, the individual is infected with Human Immunodeficiency Virus.

In various example, the fungal infection is caused by one or more fungi chosen from those of the genus Candida. Non-limiting examples of Candida fungi include, Candida albicans, Candida lusitaniae, Candida kruseii, Candida glabrata, Candida parapsilosis, Candida tropicalis, Candida guilliermondii, and the like, and combinations thereof.

In certain examples, the fungal infection is resistant to one or more antifungal agent(s) known in the art. Non-limiting examples of such antifungal agents include terbinafine, amphotericin B, candicidin, filipin, hamycin, nystatin, rimocidin, bifonazole, butoconazole, clotrimazole, econazole, fenticonazole, isoconazole, ketoconazole, luliconazole, miconazole, omoconazole, oxiconazole, sertaconazole, sulconazole, tioconazole, albaconazole, fluconazole, isavuconazole, itraconazole, psoaconazole, ravuconazole, terconazole, voriconazole, abafungin, amorolfin, butenafine, naftifine, anidulafungin, caspofungin, micafungin, ciclopirox, flucytosine, haloprogin, griseofulvin, tolnaftate, and the like, and combinations thereof.

The fungal infection may be a skin infection, a nail fungus (e.g., affecting a toenail, fingernail, or analogous structure in a non-human animal), or a combination thereof. In some examples, the skin infection is Athlete's Foot.

The methods and compositions may prevent, stabilize, or inhibit the growth of one or more fungi, or kill one or more fungi by contacting the fungi or a site susceptible to fungal growth with a composition of the present disclosure. Treating a fungal infection includes, but is not limited to, elimination of 80% or more, 90% or more, 95% or more, 99% or more, 99.5% or more, or 100% of the the fungi giving rise to the fugal infection in the individual, or complete decolonization of the fungi giving rise to the fugal infection in the individual. In various examples, a therapeutically effective amount of a composition/compositions is an amount of a composition the application of which to an individual with results in treatment of a fungal infection. The determination of an amount and/or treatment protocol that results in treating a fungal infection with one or more composition(s). In various examples, treatment with composition results in one of a reduction in viable asci, a reduction in spores and/or sporulation efficiency, a reduction in spore germination efficiency, or a combination thereof.

The administration may be a topical administration. A composition may be applied to at least a portion or all or substantially all of the exterior surface (e.g., skin) of an individual, which may be a portion of the individual exhibiting a fungal infection. By substantially all of the exterior surface (e.g., skin) of an individual, it is meant the exterior surface (e.g., skin) of an individual that may reasonably be accessed by topical administration of the composition.

Methods of the present disclosure may be used to treat various individuals. In various examples, an individual is a human or non-human mammal. Examples of non-human mammals include, but are not limited to, farm animals, such as, for example, cows, hogs, sheep, and the like, as well as pet or sport animals, such as, for example, horses, dogs, cats, rabbits, and the like. Additional non-limiting examples of individuals include, but are not limited to, laboratory test animals, such as, for example, monkeys, rabbits, [rats, mice, and the like. An individual may be a patient.

The following are non-limiting examples of procedures and protocols that are expected to be effective C. auris decolonization and environmental/human treatments. For example, a procedure and protocol for C. auris decolonization and environmental/human treatment comprises all or a portion of:
- wipe/clean entire patient with a waterless cleanser that will remove all or substantially all sweat, hygiene products, and excess moisture from the skin. Special attention may be paid to areas where fungus/yeast may accumulate (e.g., underarms, groin, under breasts, skin folds, etc.);
- shave any hair (e.g., facial hair, body hair, or both) (if possible) to keep skin as bare. Scalp ad hair should also be cleansed with the waterless cleanser;
- allow skin to completely dry (this typically takes under 1 minute to penetrate the top layer of the skin);
- remove all linen from bed including pillow cases (it is desirable that cleanable - non fabric pillows should be used in pillow cases as they do not trap moisture);
- remove all patient clothing including socks and any jewelry where yeast may accumulate underneath;
- wipe any surfaces the patient may touch with the composition(s) (e.g., bedrails, call bell, phone, mattress, etc.);
- apply the composition(s) to the patient (e.g., all over the patient's skin and scalp paying close attention to areas where fungus/yeast may accumulate);
- let mixture absorb into the skin. Do not wipe it off or dab dry. Let it sit on the skin and replace gown/clothing, sheets, blankets, etc. with new gown/clothing, sheets, blankets, etc.
- repeat this entire process (e.g., every twelve hours);
- the treatment should continue for a minimum of two weeks. After two weeks of treatment, the patient should be rescreened for C. auris.

This procedure and protocol may be completed in full every twelve hours. It is desirable that the patient remain on strict contact isolation precautions throughout the treatment process. As shared equipment such as, for example, glucometers, thermometers, blood pressure machine and cuffs, and the like are at increased risk for transmission, it is desirable that all medical equipment be patient dedicated. As water was found to spread the fungus, in various examples, the patient is not allowed to bathe/shower with any water. It is desirable that all visitors, staff and any persons entering the patient environment wear an isolation gown, gloves and shoe covers (if possible) to prevent transmission to other areas of the unit. It is desirable that the patient not be allowed in any common areas during the treatment process to prevent transmission to other patients and areas of the unit.

In an aspect, the present disclosure provides kits. A kit comprises one or more composition(s) of the present disclosure and instructions for use of the composition(s). The instructions may be instructions to carry out one or more method(s) of the present disclosure.

Kit packaging material (e.g., a physical structure housing one or more component(s) of the kit) may maintain the components sterilely, and can be made of material commonly used for such purposes (e.g., paper, corrugated fiber, glass, plastic, foil, ampules, vials, tubes, and the like, and combinations thereof). A kit may contain the one or more of the individual component(s) of a composition or compositions.

A kit optionally comprises a label or insert including a description of the composition(s) and/or the individual component(s) of the composition(s) (e.g,, type, amounts, doses, and the like), instructions for use of the composition(s) and/or the individual component(s) of the composition(s). Labels or inserts include "printed matter," e.g., paper or cardboard, or separate or affixed to a component, a kit or packing material (e.g., a box), or attached to an ampule, tube or vial containing a kit component. Labels or inserts can additionally include a computer readable medium, such as, for example, a disk (e.g., floppy diskette, hard disk, ZIP disk), optical disk such as, for example, CD- or DVD-ROM/RAM, DVD, MP3, magnetic tape, or an electrical storage media such as, for example, RAM and ROM or hybrids of these such as magnetic/optical storage media, FLASH media or memory type cards.

The steps of the methods described in the various embodiments and examples disclosed herein are sufficient to carry out the methods of the present invention. Thus, in an embodiment, a method consists essentially of a combination of the steps of the methods disclosed herein. In another embodiment, a method consists of such steps.

The following Statements are examples of compositions and methods of the present disclosure:
Statement 1. A composition comprising, consisting essentially of, or consisting of: optionally, copper sulfate; a copper metal component; and one or more organic antifungal component(s). A composition may not comprise water (e.g., are substantially anhydrous or anhydrous) and/or Group I cation salt(s).
Statement 2. A composition according to Statement 1, wherein the copper metal component is a copper powder. It may be desirable that the copper powder have consistency and/or one or more of the flow and/or rheological characteristic(s) of cornstarch or talcum powder.
Statement 3. A composition according to Statement 1 or 2, wherein the one or more organic antifungal component(s) is/are chosen from: tea tree oil, jojoba oil, and the like, and combinations thereof.
Statement 4. A composition according to any one of the preceding Statements, wherein the copper sulfate is present at 55 to 68 percent by weight (which may be based on the total weight of the composition); and/or the copper metal component(s) is/are present at 30 to 44 percent by weight (which may be based on the total weight of the composition) and/or the organic antifungal component(s) is/are present at 0.5 to 2 percent by weight (which may be based on the total weight of the composition).
Statement 5. A composition according to any one of the preceding Statements, further comprising, consisting essentially of, or consisting of one or more of the following: one or more carbon source(s) (e.g., activated charcoal and the like); and/or one or more essential oil(s). It may be desirable that the carbon source(s) is/are nitrate inhibitors. It is desirable that the essential oil(s) exhibit an antifungal property. In various examples, 0.01 to 1 percent by weight (which may be based on the total weight of the composition) of the copper sulfate is replaced by one or more essential oils(s).
Statement 6. A composition according to any one of the preceding Statements, wherein the composition comprises or is: 10 grams copper sulfate, 5 grams copper powder, 100mL jojoba oil, and 50 mL tea tree oil; 10 mL tea tree oil, 8 grams copper sulfate, and 5 grams copper powder; 10 grams copper sulfate, 6 grams copper powder, 180 mL jojoba oil; 10 grams copper sulfate, 5 grams copper powder, 100mL jojoba oil, 50 mL tea tree oil, 0.5 mg of fermented roasted garlic oil; or 5 grams copper powder; 50 mL tea tree oil, 0.5 mg of activated charcoal.
Statement 7. A composition according to Statement 5 or 6, wherein the carbon source(s) is/are chosen from: carbon powders (such as, for example, activated charcoals and the like), and combinations thereof.
Statement 8. A composition according to any one of Statements 5-7, wherein the essential oil(s) comprise or is/are chosen from: lavender oil, white sage oil, marjoram, neroli, patchouli, peppermint, palmarosa, myrrh, thyme, cassia, savory, lemongrass, citronella, coriander seed, eucalyptus, sweet fennel, ho wood, kanuka, rosemary, rosewood, vertiver, oregano, and the like, and combinations thereof. Without intending to be bound by any particular theory, it is considered that addition of essential oil(s) may provide a composition with desirable scent or smell.
Statement 9. A composition according to any one of Statements 5-9, wherein the carbon source(s) is/are present at 0.5 to 1 percent by weight (which may be based on the total weight of the composition); and/or the essential oil(s) is/are present at 0.01 to 1 percent by weight (which may be based on the total weight of the composition).
Statement 10. A composition according to any one of the preceding Statements, wherein the composition is a suspension.
Statement 11. A composition according to one of the preceding Statements, wherein the composition exhibits an antifungal property.
Statement 12. A composition according to any one of the preceding Statements, wherein the composition inhibits or prevents the growth of and/or kills one or more fungi. Non-limiting examples of fungi include Candida (such as, for example, c. auris, c. albicans, c. parapsilosis, c. lusitaniae, c. kruseii, c. glabrata, c. tropicalis, c. guilliermondii, and the like, and combinations thereof) and the like and combinations thereof.
Statement 13. A composition according to any one of the preceding Statements, wherein the composition is used to treat an individual (e.g., an individual having or suspected of having a fungal infection); or medical equipment. Non-limiting examples of medical equipment include: medical instruments, medical devices, beds and related equipment, such as, for example, bed frames, bed rails, call bells, and the like, medical clothing, such as, for example, gowns, caps, masks, gloves, socks, shoe covers, and the like, bedding materials, such as for example, mattresses, sheets, blankets, pillows, pillow cases, and the like, phones, and the like (e.g., anything typically found in a patient's room).
Statement 14. A method of inhibiting or preventing the growth of and/or killing one or more fungi comprising, consisting essentially of, or consisting of: contacting at least a portion of or all of a surface with one or more composition(s) of any one of Statements 1-13. One skilled in the art would appreciate that the amount of composition(s) used will depend on the size of the surface (e.g., patient, medical device, medical equipment, food handling equipment, or the like). As an illustrative example, approximately twelve ounces per application is contacted twice a day.
Statement 15. A method according to Statement 14, wherein the surface is part or all of an article of medical equipment (e.g., all or part of the exterior surface and/or interior surface of the medical equipment).
Statement 16. A method according to Statement 14 or 15, wherein the surface is at least a portion or all of the skin of a human or non-human mammal. This may be referred to as a topical treatment. One skilled in the art would appreciate that the amount of composition(s) used will depend on the size of the patient. As an illustrative example, approximately twelve ounces per application is contacted twice a day.
Statement 17. A method according to Statement 16, wherein at least a portion of or all of the composition is absorbed into to the skin of the individual. One skilled in the art would appreciate that the absorption time will vary depending on the patient. As an illustrative example, typically, the absorption takes about an hour.
Statement 18. A method according to Statement 14-17, wherein contacting comprises two or more individual contacting treatments.
Statement 19. A method according to Statement 18, wherein the individual contacting treatments are repeated every 10 to 14 hours (e.g., about every 12 hours).
Statement 20. A method according to Statement 18 or 19, wherein the contacting is carried out over a period of 14 days or less. The patent may be retested after conclusion of the contacting and, if appropriate, the contacting can be repeated.
Statement 21. A method according to any one of Statements 16-20, wherein substantially all or all of the human or non-human animal is hairless or substantially hairless. For example, substantially all or all of the hair, if present, of the human or non-human animal is removed (e.g., mechanically or chemically) prior to contact with the composition(s).
Statement 22. A method according to any one of Statements 16-21, wherein the human or non-human animal is in contact isolation during the contacting and, optionally, for a desired period of time after the contacting(s).
Statement 23. A method of any one according to Statements 16-22, wherein the human or non-human animal (e.g., the contacted surface(s) of the human or non-human animal) is not exposed to liquid water during the contacting and, optionally, for a desired period of time after the contacting(s). Non-limiting examples of exposure to liquid water include bathing and the like.
Statement 24. A method according to any one of Statements 16-23, further comprising, consisting essentially of, or consisting of comprising contacting the human or non-human animal with a waterless/water-free composition (e.g., a cleanser). Without intending to be bound by any particular theory, it is considered that the waterless/water-free composition removes at least a portion of or all of the sweat, product, or moisture, or a combination thereof, from the surface (e.g., skin) of the human or non-human animal.
Statement 25. A method according to any one of Statements 16-24, further comprising, consisting essentially of, or consisting of contacting one or more or all of the surface(s) that the human or non-human animal can contact (e.g., are in proximity to or in the room in which the human or non-human animal are treated) is contacted with a composition of any one of Statements 1-13.
Statement 26. A method according to any one of Statements 16-25, wherein one or more or all of the medical equipment used during the contacting is contacted with a composition of any one of Statements 1-13.
Statement 27. A method according to any one of Statements 14-26, further comprising, consisting essentially of, or consisting of testing for the presence of the one or more fungi after one or more of the contacting(s). The testing can be carried out by methods known in the art. The testing may be carried out by culture-based methods or culture-independent methods. For example, the testing is carried out by protocols published (e.g., for testing for Candida auris) by the Center for Disease Control (CDC) and the New Jersey State Department of Health. Examples of testing protocols are available at www.CDC.gov. and NJDOH. An example of a testing method is provided herein.

The following examples are presented to illustrate the present disclosure. They are not intended to be limiting in any matter.

### EXAMPLE 1

This example provides a description of antifungal compositions and methods of treating and/or decolonizing fungal infections of the present disclosure.

**Table 1. Testing results.**

| **PLATE #** | **INGREDIENTS** | **WEIGHT/VOLUME** | **TEMP INCUBATED** | **RESULT** |
|---|---|---|---|---|
| 1 | • COPPER SULFATE | • 9 GRAM | 36 DEGREE C | NO GROWTH & KIL LED/INHIBITED FURTHER GROWTH |
| | • COPPER POWDER | • 5 GRAM | | |
| | • JOJOBA OIL | • 3 MLS | | |
| 2 | • COPPER SULFATE | • 4 GRAM | 36 DEGREE C | NO GROWTH & KILL ED/INHIBITED FURTHER GROWTH |
| | • COPPER POWDER | • 10 GRAM | | |
| | • TEA TREE OIL | • 1.5 MLS | | |

Process for composition preparation:
- copper sulfate and copper powder ground to fine consistency;
- weigh and add each ingredient;
- mix vigorously;
- mixture was used as a suspension (if needed, shake or mix immediately before application)

### EXAMPLE 2

This example provides a description of antifungal compositions and methods of treating and/or decolonizing fungal infections of the present disclosure.

Ten Chomagar Candida plates were tested, with the intention of suppressing Candida auris growth. Two days after testing began, results were recorded and modifications made to select plates (see Graph #1). Temperature for all specimens was maintained at 36 C°.

**Graph #1**

| | | |
|---|---|---|
| - Plate #1 | - C. auris live culture | - Growth as expected |
| | - (Used as a positive control) | |
| - Plate #2 | - C. auris live culture | - Approx. 30% less growth |
| | - 100 uL tea tree oil | |
| | | - (compared to control) |
| - Plate #3 | - C. auris live culture | - Approx. 60% less growth |
| | - 100 uL tea tree oil | |
| | - 5gm Fine Copper powder | - (compared to control) |
| - Plate #4 | - C. auris live culture | - Approx. 50% less growth |
| | - 5gm fine copper powder | |
| | | - (compared to control) |
| - Plate #5 | - C. auris live culture | - Approx 20% less growth |
| | - 50 uL lavender oil | |
| | - 50 uL clary sage oil | - (compared to control) |
| - Plate #6 | - C. auris live culture | - Approx 60% less growth |
| | - 50 uL lavender oil | |
| | - 50 ul clary sage | - (compared to control) |
| | - 5gm fine copper powder | |
| - Plate #7 | - C. auris live culture | - Excessive growth |
| | - 10gm copper sulfate crystals | - (compared to control) |
| | - 1.5 ml deionized water | |
| - Plate #8 | - C. auris live culture | - Excessive growth |
| | - 10gm copper sulfate crystals | - (compared to control) |
| | - 0.5 ml hydrogel | |
| - Plate #9 | - C. auris live culture | - Excessive growth |
| | - 5gm Fine Copper Powder | - (compared to control) |
| | - 0.5 ml hydrogel | |
| - Plate #10 | - C. auris live culture | - 2 colonies noted |
| | - 10gm copper sulfate crystals | - (compared to control) |
| | - 600 uL tea tree oil | |

Three days after testing began, plates in Graph #2 were read and results documented.

**Graph #2**

| | | |
|---|---|---|
| Plate #3 | Added: 150 uL Ammonium Lactate 5gm Fine Copper Powder | Unable to read - contents exploded |
| Plate #4 | Added: 150 uL Ammonium Lactate | Unable to read - contents exploded |
| Plate #6 | Added: 50 uL Tea Tree Oil 1 inch square of Copper Foil | No new growth noted Growth under foil square was destroyed |
| Plate #9 | Added: 50 uL Tea Tree Oil | Slowed growth |
| Plate #10 | Added: 5gm Fine Copper Powder | No new growth - 2 colonies were destroyed |

Two additional compositions were plated. One day and again two days after testing began, results were read and documented. (Graph #3)

**Graph #3**

| | | |
|---|---|---|
| Plate #11 | C. auris Live Culture | Unable to read -Exploded |
| | 50 uL Tea Tree Oil | |
| | 10 gm Copper Sulfate | |
| | 150 uL Ammonium Lactate | |
| Plate #12 | C. auris Live Culture | No growth noted |
| | 50 uL Tea Tree Oil | |
| | 10 gm Copper Sulfate | |
| | 5gm Copper Powder | |

Five additional compositions were plated. Four days after testing began, results were read and documented. (Graph #4)

**Graph #4**

| | | |
|---|---|---|
| Plate #1 | C. auris Live Culture | No growth noted |
| | 5 gm Fine Copper Powder | *a wet mount was done to check for growth - no growth noted |
| | 50 uL Tea Tree Oil | |
| Plate #2 | C. auris Live Culture | Questionable minimal growth noted |
| | 5 gm Fine Copper Powder | |
| | 50 uL Tea Tree Oil | *Two days after plating a subculture was plated - No growth |
| | 0.5 Capsule Activated | |
| | Charcoal | |
| Plate #3 | C. auris Live Culture Half Plate Salted Seaweed Half Plate Copper Foil | Excessive growth - plate discarded |
| Plate #4 | C. auris Live Culture 2 inch square Copper Foil | Normal growth noted |
| Plate #5 | C. auris Live Culture | No Growth noted |
| | 0.5 mg of Roasted Garlic | |
| | 50 uL Tea Tree Oil | Two days after plating: re-check - no growth |
| | 10 gm Copper Sulfate | |
| | 5 gm Fine Copper Powder | |
| | 100 uL Jojoba Oil | wet mount done - no growth |

Two additional compositions were plated. Six days after testing began, results were read and documented. (Graph #5)

**Graph #5**

| | | |
|---|---|---|
| Plate #1 | C. auris Live Culture | No growth |
| | 180 uL Tea Tree Oil | |
| | 8 gm Copper Sulfate | wet mount done - no growth |
| | 5 gm Fine Copper Powder | |
| | | Seven days after plating: subculture taken - no growth |
| Plate #2 | C. auris Live Culture | No growth |
| | 180 uL Jojoba Oil | |
| | 8 gm Copper Sulfate | wet mount done - no growth |
| | 5gm Fine Copper Powder | Seven days after plating: subculture taken - no growth |

Two additional compositions were plated. Four days after testing began, results were read and documented. (Graph #6)

**Graph #6**

| | | |
|---|---|---|
| Plate #1 | C. auris Live Culture | No growth |
| | 10 gms Copper Sulfate | |
| | 6 gms Fine Copper | |
| | 180 uL Jojoba Oil | |
| Plate #2 | C. auris Live Culture | Questionable growth |
| | 8.69 gm Copper Sulfate | |
| | 5 gm Copper Powder | Subcultured, no growth |
| | 90 uL Tea Tree Oil | |

It was concluded that the compound mixture of fine copper powder, copper sulfate and antifungal essential oil (tea tree oil or jojoba oil) was effective in inhibiting and suppressing the growth of Candida auris. Additives such as lavender oil, cleary sage oil, and any other fragrant essential oil that has antifungal properties could be added for scent.

Any compound mixture that contained water or water based products (hydrogel) or the salted seaweed increased the growth and spread of the fungus around the plate. It was concluded that C. auris thrived with water and sodium.

This compound was not tested on humans with active C. auris colonizations or the environment. These tests were conducted in a controlled laboratory setting.

Process for preparation of compositions:
- grind copper powder finely;
- grind copper sulfate crystals finely;
- weigh and add each ingredient to a sterile cup using a pipette;
- add any optional components;
- mix vigorously. Mixture should remain as a suspension for use. (If needed, shake or mix immediately before application to skin).

Materials and Methods
- Equipment Used:
   - Precision weighing instrument, Ohaus Scale, model Adventurer Pro AV413CN
   - Thermo Scientific Biological Safety Cabinet, model 1375
   - Thermo Scientific 37 degree C Incubator, model 51026067
- Material Used:
- *(Chemicals*/*Reagents)*
   - BDBBL prepared plated media Chomagar Candida
   - Copper sulfate (Active Ingredient)
   - Copper powder (Active Ingredient)
   - Roll of Copper foil
   - Ammonium lactate
   - Skintegrity Hydrogel
   - Nature's Way activated charcoal capsules, 400mg capsules
   - Fernented black garlic
   - Salted, dried seaweed
- Other Known Antifungals Used:
   - Maple Holistics 100% Pure Tea Tree Essential Oil (Active Ingredient)
   - Desert Essence 100% Pure Jojoba Oil (Active Ingredient)
   - Aura Cacia Lavender 100% Pure Essential Oil (Optional Additive for scent)
   - Aura Casia Clary Sage 100% Pure Essential Oil (Optional Additive for scent)
- Live Cultures:
   - Live Candida auris cultures, cultured on Chromagar Media
- Other Materials Used:
   - Sterile plastic cups
   - Sterile cotton swabs
   - Nitrile gloves
   - Fisherbrand plastic pipette tips
   - Copan Diagnostics inoculating loops
   - Super Sani-Cloth Disinfectant Wipe
   - Cell Phone Camera (for photo purposes)

Although the present disclosure has been described with respect to one or more particular embodiment(s) and/or example(s), it will be understood that other embodiment(s) and/or example(s) of the present disclosure may be made without departing from the scope of the present disclosure.

## Claims

1. A composition comprising:
optionally, copper sulfate;
a copper metal component; and
one or more organic antifungal component(s).

2. The composition of claim 1, wherein the copper metal component is a copper powder.

3. The composition of claim 1 or 2, wherein the one or more organic antifungal component(s) is/are chosen from:
tea tree oil, jojoba oil, and the like, and combinations thereof.

4. The composition of any one of the preceding claims, wherein
the copper sulfate is present at 55 to 68 percent by weight based on the total weight of the composition; and/or
the copper metal component(s) is/are present at 30 to 44 percent by weight based on the total weight of the composition and/or
the organic antifungal component(s) is/are present at 0.5 to 2 percent by weight based on the total weight of the composition.

5. The composition of any one of the preceding claims, further comprising one or more of the following:
one or more carbon source(s); and/or
one or more essential oil(s),
wherein the carbon source(s) may be present at 0.5 to 1 percent by weight based on the total weight of the composition and/or the essential oil(s) may be present at 0.01 to 1 percent by weight based on the total weight of the composition.

6. The composition of any one of the preceding claims, wherein the composition comprises:
10 grams copper sulfate, 5 grams copper powder, 100mL jojoba oil, and 50 mL tea tree oil;
10 mL tea tree oil, 8 grams copper sulfate, and 5 grams copper powder;
10 grams copper sulfate, 6 grams copper powder, 180 mL jojoba oil;
10 grams copper sulfate, 5 grams copper powder, 100mL jojoba oil, 50 mL tea tree oil, 0.5 mg of fermented roasted garlic oil; or
5 grams copper powder; 50 mL tea tree oil, 0.5 mg of activated charcoal.

7. The composition of claim 5 or 6, wherein the carbon source(s) is/are chosen from:
carbon powders and combinations thereof.

8. The composition of any one of claims 5-7, wherein the essential oil(s) comprise or is/are chosen from:
lavender oil, white sage oil, marjoram, neroli, patchouli, peppermint, palmarosa, myrrh, thyme, cassia, savory, lemongrass, citronella, coriander seed, eucalyptus, sweet fennel, ho wood, kanuka, rosemary, rosewood, vertiver, oregano, and the like, and combinations thereof.

9. The composition of any one of the preceding claims, wherein the composition is a suspension.

10. A method of inhibiting or preventing the growth of and/or killing one or more fungi comprising:
contacting at least a portion of or all of a surface with one or more composition(s) of any one of claims 1-9.

11. One or more composition(s) of any one of claims 1-9 for use in a method of inhibiting or preventing the growth of and/or killing one or more fungi, the use comprising:
contacting at least a portion of or all of a surface with the one or more composition(s) of any one of claims 1-9.

12. The method of claim 10, wherein the surface is part or all of an article of medical equipment, and, optionally, one or more or all of the medical equipment used during the contacting is contacted with a composition of any one of claims 1-9.

13. Theone or more composition(s) for use of claim 11, wherein the surface is at least a portion or all of the skin of an individual, which may be a human or non-human mammal, and, optionally, at least a portion of or all of the composition is absorbed into to the skin of the individual.

14. The method of claim 10 or 12 or the one or more composition(s) for use of claim 11 or 13, wherein contacting comprises two or more individual contacting treatments, wherein the individual contacting treatments may be repeated every 10 to 14 hours (e.g., about every 12 hours), and/or the contacting is carried out over a period of 14 days or less.

15. The one or more composition(s) for use of any one of claims 11, 13 or 14, wherein substantially all or all of the human or non-human animal is hairless or substantially hairless and/or the human or non-human animal is in contact isolation during the contacting and, optionally, for a desired period of time after the contacting(s) and/or the human or non-human animal (e.g., the contacted surface(s) of the human or non-human animal) is not exposed to liquid water during the contacting and, optionally, for a desired period of time after the contacting(s); and / or
further comprising contacting the human or non-human animal with a waterless/water-free composition (e.g., a cleanser) and/or contacting one or more or all of the surface(s) that the human or non-human animal can contact (e.g., are in proximity to or in the room in which the human or non-human animal are treated) is contacted with a composition of any one of claims 1-9, and/or further comprising testing for the presence of the one or more fungi after one or more of the contacting(s).
